# EUROPEAN PATENT APPLICATION

(11) **EP 1 424 391 A1**
(43) Date of publication of application: **02.06.2004**
(21) Application number: 02760575.7
(22) Date of filing: 07.08.2002
(51) Int. Cl.: C12N 15/09, C12Q 1/68, C12Q 1/02

(54) **GENE PANEL PARTICIPATING IN LIVER ASTROCYTE ACTIVATION**

(30) Priority: 08.08.2001 JP 2001240974
(71) Applicant: Ajinomoto Co., Inc., Tokyo 104-8315 (JP)
(72) Inventor: YOKOYA, Fumihiko, C/O AJINOMOTO Co., Inc., Kawasaki-shi, Kanagawa 210-8681 (JP); TAKAHARA, Yoshiyuki, C/O AJINOMOTO Co., Inc., Kawasaki-shi, Kanagawa 210-8681 (JP); OKUTSU, Tomohisa, C/O AJINOMOTO Co., Inc., Kawasaki-shi, Kanagawa 210-8681 (JP)
(74) Representative: HOFFMANN - EITLE
(86) International application number: PCT/JP2002/008079
(87) International publication number: WO 2003/014345

(57) **Abstract**

A gene panel comprising genes each showing, in hepatic stellate cells, a varied expression level in a hepatic stellate cell activation state compared with a level in a normal state, comprising the steps of:
(a) measuring expression levels of various genes in the hepatic stellate cells, which have been separated from a model animal in the normal state, in the resting state and the expression levels of the genes in the hepatic stellate cells in the activation state; and
(b) identifying the genes showing the increased expression level in the activation state.

## Description

### Technical Field

The present invention relates to a gene panel comprising genes each showing an increased expression level in hepatic stellate cells in accordance with activation of the hepatic stellate cells compared with that in a normal state, a method for producing therefor, and a use of the gene panel. The present invention is useful in the field of diagnosis, pharmaceutical, or the like.

### Background Art

Hyperplasia and accumulation of hepatic connective tissues lead to circulatory disturbance of the liver. It is considered that this also causes hepatocyte injury, thereby forming a vicious cycle in which further hyperplasia and accumulation occur, resulting in liver disease accompanying liver cirrhosis and hepatic fibrosis in a hepatic fibrosis model animal. The liver consists of parenchymal cells (hepatocyte) and non-non-parenchymal cells (hepatic stellate cells, Kupffer cells, sinusoidal endothelial cells, and Pit cells), and the hepatic connective tissue is constituted of extracellular matrix and cells localized therein. Activation and transformation of hepatic stellate cells, which are stroma producing cells in connective tissues, promote hyperplasia and accumulation of the connective tissues. It is known that the hepatic stellate cell of normal liver (hereinafter referred to as (quiescent) stellate cell in a resting state) produces the extracellular matrix in a small amount, is transformed into a myofibroblast-like cell in accordance with the activation and synthesizes a large amount of extracellular matrix together with the increase of the cells.

Therefore, there is required a medical care in which the activation of the hepatic stellate cells in a patient suffering from liver disease with hepatic fibrosis such as cirrhosis is suppressed to thereby alleviate hyperplasia and accumulation of the connective tissue. Thus, it can be said that screening for drugs which inhibit the activation of the hepatic stellate cells is important. Despite the above requirement, there has not been known an effective method of screening drugs inhibiting the activation of hepatic stellate cells.

Once hepatic cells have come to necrosis by hepatic disease, the hepatic stellate cells which have been in a resting state till then are activated by humoral factors derived from the hepatic necrosis cells, cytokines being paracrine-secreted from the activated Kupffer cells at inflammatory local sites or invaded inflammatory cells, and further autocrine-secretion by the activated hepatic stellate cells. Note that; a plurality of factors are considered to cause the activation of the hepatic stellate cells, but it is generally uncertain what timing they act at. It is thus important to study a plurality of essential genes acting for the hepatic stellate cell activation fort the purpose of grasping the whole image of the hepatic stellate cell activation, because a plurality of genes are considered to cause the hepatic stellate cell activation. No drugs have ever been produced from screening of the drugs suppressing hepatic stellate cell activation. There has been reported gene screening in which the observation of expression changes during hepatic stellate cell activation is used as index. However, in the above gene screening, the observation of expression changes is carried out on at most several kinds of genes.

### Disclosure of the Invention

The present invention has been made in view of the above and it is an object of the invention to provide a gene panel comprising genes showing a change in an expression level in a state of hepatic stellate cell activation, compared with a resting state of hepatic stellate cells, and a method of screening a drug that inhibits the activation of hepatic stellate cells.

The present inventors considered that, for inhibiting the activation of hepatic stellate cells, a behavior of genes related to the activation of hepatic stellate cells should be made similar to a gene expression pattern of hepatic stellate cells in the resting state. In addition, the invention has been completed as a result of investigating expression profiles of various genes in the activation of hepatic stellate cells and obtaining information about the expression of genes related to the hepatic stellate cells using an activation model in a primary culture system of hepatic stellate cells.

More specifically, the present invention is as follows.
(1) A gene panel comprising names and gene expression profiles of genes each showing, in hepatic stellate cells, an increased expression level in an activation state compared with a level in a resting state.
(2) The gene panel as described in the item (1) or (2), in which the increased expression level of the gene corresponds to a difference of an expression level in a model animal having liver cirrhosis and hepatic fibrosis with an expression level in a normal state in a model animal.
(3) The gene panel as described in the item (1) or (2), in which the expression profile comprises a time-varying expression profile in the hepatic stellate cells.
(4) The gene panel as described in the item (2) or (3), in which the model animal is a rat.
(5) The gene panel as described in any one of the items (1) to (4), further comprising an expression profile of each of at least 5 kinds of genes among 105 kinds of genes represented as Nos. 1 to 105 listed in Tables 1 to 4.
(6) A method of producing a gene panel comprising genes each showing, in hepatic stellate cells, a varied expression level in a hepatic stellate cell activation state compared with a level in a normal state, comprising the steps of:
   (a) measuring expression levels of various genes in the hepatic stellate cells in the resting state and the expression levels of the genes in the hepatic stellate cells in the activation state;
   (b) comparing the expression levels with each other; and
   (c) identifying the genes showing the increased expression level in the activation state.
(7) The method as described in the item (6), wherein the expression levels of the genes in the hepatic stellate cells activation state are analyzed in time course in the step (a).
(8) The method as described in the item (6) or (7), wherein the expression levels of the gene are analyzed by a gene chip method.
(9) A method for screening a drug related to hepatic stellate cell activation, comprising the steps of administering the drug to a model animal or hepatic tissues or cells, and profiling expressions of genes constructing the gene panel as described in the item (1).

Hereinafter, the present invention will be described in detail.

### <1> Gene panel of the present invention

The gene panel of the present invention is a gene panel comprising the names of the genes, each of which shows an increase in its expression level in hepatic stellate cells in an activation state, compared with the resting state thereof; and their respective gene expression profiles.

The gene panel of the present invention can be produced in the steps of:
(a) measuring expression levels of various genes in a hepatic stellate cell in a resting state and expression levels of the genes in hepatic stellate cells in an activation state;
(b) comparing the expression levels with each other; and
(c) identifying genes showing an increased expression level in the activation state.

The functions of hepatic stellate cells include the metabolism and storage of vitamin A, the production of extracellular matrix, the production of cytokines, contractility, and the transformation to myofibroblast cells. These functions are considered to be closely related to hepatic fibrosis. The term "hepatic stellate cells in an activation state (synonymous with activated hepatic stellate cells or active-type hepatic stellate cells)" means hepatic stellate cells in a state of being transformed from hepatic stellate cells in the resting state of the normal liver to myofibroblasts under various stimuli. The cell transformed to the myofibroblast shows an increase in proliferative property, a decrease in the content of vitamin A, and an increase in contractility. In addition, the cell produces an extracellular matrix that mainly contains type I collagen. Furthermore, the synthesis of an extracellular matrix catabolic enzyme such as collagenase or gelatinase slows down, so that the extracellular matrix can be increased and accumulated.

The activated hepatic stellate cells have been known for causing similar transformation in isolated hepatic stellate cells. In hepatic diseases such as hepatitis (J hepatology 1996 Mar 24, (3):301-7 Guido M liver stellate cells in chronic viral hepatitis: the effect of interferon therapy), cirrhosis (American Journal of pathology 1990 July 137(1) Stefano M et al. Cellular Localization of Type I, III, and IV Procollagen Gene transcripts in Normal and Fibrotic Human liver), and NASH (nonalcoholic steatohepatitis)(Hum Pathol 2000 July 31(7)822-8, Washington K, et al Hepatic stellate cell activation in nonalcoholicsteatohepatitis and fatty liver.), an increase in number of the active-type hepatic stellate cells is observed as connective tissues proliferate and accumulate in the liver with its sustained inflammatory.

The term "expression level of a gene" is synonymous with the expression amount, expression intensity, or expression frequency of a gene, which is analyzed with the production amount of a translation product corresponding to the gene, the activity of the translation product, or the like.

The expression level of the gene can be measured using any method generally used for the analysis of gene expression. Preferable methods include a gene chip method, a gene microarray method, and a gene macroarray method. Each of them arranges and attaches gene fragments on any plate (typically, slide glass). It allows the chip to hybridize with fluorescence-labeled mRNA to quantify and specify the mRNA.

Alternatively, other methods of analyzing the gene expression include an ATAC-PCR method (Nucleic Acids Research 25,4694-4696(1997)), a Body Map method (Gene, 174, 151-158(1996)), the serial analysis of gene expression (SAGE) (US527,154B, US544,861B and EP0761,822A), and a MAGE (Micro-analysis of Gene Expression) (JP 2000-232888A).

The ATAC-PCR method will be outlined as follows. At first, a double-stranded DNA is prepared from cDNA synthesized with a 5'-biotinylated oligo-dT primer and is then digested with a given restriction enzyme (the example using *MboI* will be described herein). Subsequently, adaptors having a sequence common to the portions cleaved with the restriction enzyme (preparing six adaptors having different lengths) and the double-stranded DNA cut out with the restriction enzyme *MboI* are ligated with each other by a DNA ligase. Furthermore, among these six different adaptors, three of them are coupled to the respective control cDNAs (cDNAs prepared from hepatic stellate cells in the activation state) and then mixed at a ratio of 10:3:1. The remaining three adaptors are coupled to cDNAs prepared from rat hepatic stellate cells cultured for 4 hours, 3 days, and 7 days in non-coating plastic petri-dishes, respectively.

After mixing the respective ligation products, the 3'-fragment of the double-stranded cDNA is recovered using streptavidin-coated beads to perform a competitive RT-PCR by using primers having sequences common to the respective adaptors. The PCR product is analyzed with the ABI PRISM 3700 DNA Analyzer. This system is able to isolate fragments for each of different lengths thereof by capillary electrophoresis, so that the intensity of fluorescence can be detected in proportion to the expression amount.

The Body Map method will be outlined as follows: cDNA is prepared from mRNA using the poly-T sequence of a vector as a primer such that the poly-A tail of the 3' end of the mRNA is coupled to the vector's poly-T sequence. Furthermore, the cDNA is cut out with the restriction enzyme *Mbo*I. As the cDNA has an *MBo*I site every 300 base pairs on average, the cDNA on the vector will be cleaved every 300 base pairs on average. At this time, the cDNA proximate to the poly-A tail still remains coupled to the vector. The vector having this cDNA fragment is cyclized and then introduced in *E. coli* to prepare a cDNA library. About 1,000 clones are arbitrary selected from the library and the nucleotide sequences having 300 base pairs on average are defined for the respective clones. Among these sequences, clones are sorted out into groups such that each group includes clones having the same sequence. Then, these sequence kinds and the expression frequencies of the respective sequences are investigated and calculated to obtain a gene expression profile. Each cDNA sequence is subjected to a search for the homology thereof to a data bank (the BLAST search). When the clone has the same sequence as that of a known gene, the name of the gene is given to the clone. When the sequence has not been registered in the data bank, it is assumed that any gene corresponding to such a sequence is not present.

For conducting the homology search with the BLAST search, the information of at least 11 base pairs will be required. There are about one million kinds of a sequence consisting of 10 bases, exceeding the number of gene types (100,000 types) expected to be present in human beings. In other words, the information about 11 base pairs makes it possible to specify the gene having the sequence, allowing a gene expression profile analysis. Therefore, for more efficiently conducting the gene expression profile analysis with the Body Map that requires a great number of sequences, cDNA fragments of about 300 base pairs in the Body Map are made into shorter fragments of 11 or more base pairs (referred to as "tags"). Then, these fragments are linked together in large quantity and then inserted into a vector. Consequently, a linked tag library is prepared and then about 1,000 clones are arbitrary selected in the same manner as that of the Body Map. Determining the DNA sequence of the linked tag can be expected to obtain more information about gene expression in the same procedure as that of the Body Map. The tag represents a gene sequence and the appearance frequency of the tag corresponds to the expression frequency of the gene thereof. Generally, the length of a DNA sequence which can be read out per sequencing is about 600 base pairs, so that the DNA sequences of about 50 tag DNAs can be read out at the maximum through one sequencing. In other words, the gene expression profile analysis can be performed with an efficiency about 50 times as high as that of the Body Map method.

The SAGE method is a gene expression profile analysis based on the above ideas. The SAGE method will be conducted as follows: cDNA is prepared using poly-T where the 3'-end thereof is coupled to biotin is used as a primer. Similarly to the Body Map, cDNA as a primer is prepared using poly-T having the 3'-end coupled to biotin and then the cDNA is cut out by a restriction enzyme such as *Mbo*I (referred to as an "anchoring enzyme"). After that, cDNA fragments containing the biotin-coupled 3'-ends are adsorbed on avidin beads, followed by dividing the beads into two groups. Then, one of two linkers (A or B) is coupled to the cDNA fragment (about 13 bp) adsorbed on one of bead groups. In each linker, the site of a Class-II restriction enzyme such as *Bsm*FI (referred to as a "tagging enzyme") is included in advance. The tagging enzyme cuts the cDNA fragment out of the beads and flattens the cleavage site thereof to link the tag coupled to the A linker with the tag coupled to the B inker. It is referred to as a "ditag". The ditag is amplified by PCR using a primer that recognizes both the A linker and the B linker. The great number of amplified ditags are coupled together and then incorporated into a vector, followed by sequencing. About 50 tag sequences can be read out at the maximum through one sequencing. The frequency of gene expression can be derived from the compiled information about the tag sequence.

The MAGE method is an improved method of the above methods. The method is capable of analyzing the expression frequency of a gene efficiently with high precision by preparing cDNA from mRNA using a vector primer having a poly-T sequence, tagging the cDNA sequence on the vector, ligating the resulting tags through the intermediation of a sequence capable of recognizing the end of the tag to form a concatamer, and analyzing the nucleotide sequence of the concatamer.

In the present invention, the method is not specifically limited as far as it can analyze the expression level of a gene. Any method presently known in the art or method to be developed in future can be adopted. Among the above methods, particularly preferable methods include a gene chip method, a gene microarray method, and an ATAC-PCR method.

In the present invention, the analysis of gene expression may be performed depending on the result obtained by a single method or may be performed by combining the results obtained from two or more methods. Even though the single method allows the analysis, the combination of two or more methods allows a more precise analysis. Specifically, the coefficient of correlation between the results of two or more methods, for example, between a result obtained by the gene chip method and a result obtained by the ATAC-PCR method, is calculated. Then, the gene having a correlation coefficient above a certain level is evaluated as being changed in the expression level.

Various gene chips of humans and animals such as mice and also various gene micro- and macro-arrays are commercially available, so that the present invention may adopt one of them.

In the present invention, a change in expression level of a gene can be analyzed by measuring the expression levels of various genes in hepatic stellate cells in the resting state and the expression levels of these genes in the hepatic stellate cells in the activation state and making a comparison between the respective expression levels.

The expression level of the gene in the hepatic stellate cells in the resting state can be analyzed by measuring the expression level of the gene in the hepatic stellate cells directly after isolating from a normal liver.

On the other hand, the expression level of the gene in the hepatic stellate cells in the activation state can be analyzed by, for example, incubating hepatic stellate cells isolated from a normal liver as a primary culture in a non-coating plastic petri-dish, and measuring the expression level of the gene in the resulting cells. Only by incubating the hepatic stellate cells isolated from the normal liver as a primary culture in the non-coating plastic petri-dish, the cells activate themselves to show traits similar to those of the active-type hepatic stellate cells observed in the liver tissues of a clinical sample such as cirrhosis or hepatitis, for example, the decrease in accumulation of lipid droplets, and the production of extracellular matrix.

When the hepatic stellate cells are isolated from the normal liver of a rat and inoculated in a non-coated plastic petri-dish, many lipid droplets for storing vitamin A are observed in the cytoplasm of the hepatic stellate cells directly after the isolation, showing the form similar to the hepatic stellate cells of the normal liver in the resting state. After isolating the hepatic stellate cells and incubating them on a non-coating plastic petri-dish, α-smooth muscle actin, which is a marker of the activation of hepatic stellate cells, is observed 3 days after the culture, and after about 1 week, the hepatic stellate cells show the myofibroblast-like form as activated hepatic stellate cells. The activated hepatic stellate cells on the seventh day shows the form similar to that of the activated hepatic stellate cells in which lipid droplets to be observed in cirrhosis, fibrosing liver, or the like are diminished.

In addition, the expression level of a gene in hepatic stellate cells in the activation state is analyzed by measuring, for example, the expression level of the gene in the hepatic stellate cells just after isolating them from the liver of a cirrhosis /liver-fibrosing model animal. On the other hand, the expression level of the gene in the resting state is measured directly after isolating hepatic stellate cells from the liver of a normal model animal. It is preferable to analyze the expression level of the gene in time course as the hepatic stellate cells are activated.

The cirrhosis/liver-fibrosing model animal can be obtained by, for example, intraperitoneally administrating 1 ml thioacetamide in physiological saline (50 mg/ml) to a male rat twice a week for six weeks. During this period, the rat is bred in free-feeding and free-drinking.

As described above, the genes in which the expression level thereof varies in the activation state, compared with genes in the resting state, are identified.

The gene panel of the present invention includes at least the names of various genes measured as described above and the expression profiles of the respective genes, i.e., information about a change in expression level. A nomenclature for the name of a gene is not specifically limited as far as the name can be distinguished from the names of other genes. Typically, the name of the product encoded by the gene, the accession number or genetic name on a data base such as the GeneBank, the name of a probe set or the name of the gene on a gene chip, or the like is used.

In a preferred embodiment of the gene panel of the present invention, genes are classified depending on the expression level thereof after a given period of time from the isolation of hepatic stellate cells. For instance, the genes are classified into groups of an expression amount increasing after three days (at an initial stage of activation) and that increasing after seven days (the active-type hepatic stellate cells) from the isolation of hepatic stellate cells. The term remarkable used herein means that the expression amount is increased more than 3-fold as compared with that of the hepatic stellate cells in the resting state.

### <2> Screening method of the present invention

On the basis of the gene panel of the present invention, various kinds of screening is made possible by constructing a system for quantitatively or semi-quantitatively measuring the expression of a gene included in the gene panel.

For instance, it is possible to perform screening on a drug related to hepatic stellate cells by administering a drug to a hepatitis, cirrhosis, or NASH model animal or activated hepatic stellate cells and profiling the expressions of the respective genes that constitute the gene panel of the present invention. In other words, it is considered that the administration of the drug may inhibit the activation of hepatic stellate cells if the drug is one where the expression profile of each gene is similar to the expression profile in the hepatic stellate cell gene panel in the resting state.

Furthermore, a substance for inhibiting the activation of hepatic stellate cells can be also screened by screening a drug for further promoting an increase in expression of a gene in this gene panel or a drug for further promoting a reduction in expression thereof. In this case, the screening is made possible by focusing on changes in expressions of almost five kinds of genes.

The methods of profiling the expression of a gene include a DNA microarray method, a DNA macroarray method, or an ATAC-PCR method, a method using a Taqman probe, a quantitative PCR method using SYBR Green, or the like using slide glass, a nylon membrane, or the like on which fragments of genes that constitute the gene panel are fixed. The expression profiling may be performed by a single method or a combination of two or more methods.

In the following, an example of the specific screening procedures will be described.

As primary screening, isolated activated hepatic stellate cells or established activated hepatic stellate cells are treated with a screening-target drug and then RNA is prepared from the cells after a given period of time. The stellate cells isolated from the liver become active as time goes by, so that the action of the drug may be measured on the cells, which are provided as targets, at each stage of the activation, for example, an initial stage of activation (from the time immediately after the isolation to the third day of the incubation), a middle stage (from the fourth day to the sixth day of the incubation), and a later stage (from the seventh day forward of the incubation). Measuring the expression level of a gene before and after drug treatment, screening is performed for a drug where the expression profile is analogous to the gene panel of stellate cells in the resting state or a drug that inhibits the expression of a gene to be increased with the activation thereof. The gene expression patters in hepatic stellate cells after the drug treatment are sorted into groups, followed by screening a drug analogous to the profile of the gene expression panel in the resting state or a drug that inhibits the expression of a gene to be increased with the activation thereof. Grouping is performed through classification based on the expression level for a given period of time similarly to the preparation of the gene panel.

Proteins provided as gene expression products in the cells are related with each other and form networks. The networks are constructed by direct binding, establishing the relationship between an enzyme and a substrate, and controlling the transcription of a specific gene such that the protein binds to the specific site of the genome, respectively. In the activation of the hepatic stellate cells, a series of networks is actuated and then the actuation thereof actuates the subsequent series of networks. It is conceivable to provide a cascade in which the actuation actuates the subsequent network. As a result of this cascade, the hepatic stellate cells are activated and finally developed into myofibroblasts. This panel is one collectively including changes in expressions of all genes related to such a cascade. For the requirements of a hepatic stellate cell activation inhibitor, it is desired to stop the network by inhibiting the network on the upstream side or stopping the cascade by inhibiting a signal transmission around a transcription factor, via the transcription factor. From the above, the promising inhibitor may generate gene changes in this panel together. This assembly is expected to include about 10 genes related to one network in this panel, although depending on the occasion. Thus, at least five genes are expected to be changed by one inhibitor. Therefore, changes in at least five genes are involved in a judgment on the effects as primary screening.

As the conditions for culturing hepatic stellate cells, there are methods including the incubation in a non-coated plastic petri-dish, the addition of a substance that accelerates the activation of hepatic stellate cells, such as TGF-1β (tumor growth factor), and so on. It is expected that a substance for inhibiting the activation of hepatic stellate cells or a substance for inhibiting the proliferation of hepatic stellate cells will be screened.

As secondary screening, a candidate drug expected to inhibit the hepatic stellate cell activation or the hepatic stellate cell proliferation in the primary screening is administered to a pathologic model rat prepared using a hepatitis-inducing agent, a cirrhosis-inducing agent, a fatty liver-inducing agent, or the like and then the lever is enucleated from the rat. The amount of connective tissues in the liver is measured, while the RNA thereof is extracted to measure a change in gene expression. The data thereof is compared with the data of a change in gene expression which is measured while measuring an amount of the liver connective tissues of the rat administered with no drug to evaluate the effects of the drug on the proliferation or accumulation of liver connective tissues.

It is possible to carry out the above screening using an experimental animal other than a rat. In this case, it is preferable to perform the screening by rebuilding a gene panel with respect to a homolog corresponding to a rat gene.

As described above, the gene panel of the present invention is considered to be useful in screening of an effective drug for the activation of hepatic stellate cells. As a result, in combination with the resulting drugs, supposedly, it is also possible to create a more effective remedy for the hepatic fibrosis.

### Brief Description of the Drawing

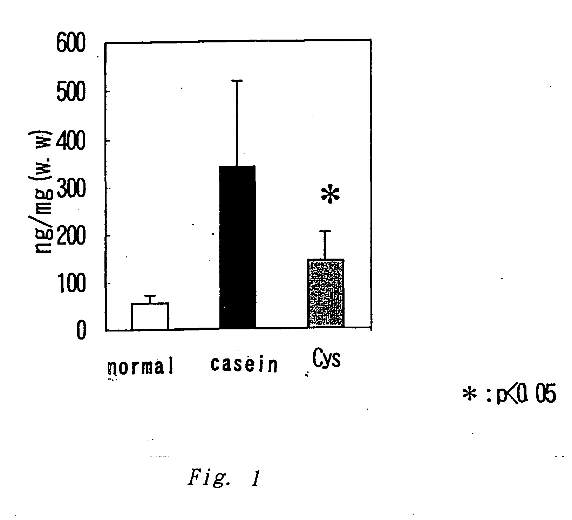

Fig. 1 shows an amount of hydroxyproline (Hyp) in the liver of a rat fed with casein or L-cysteine.

### Best Mode for carrying out the Invention

Hereinafter, the present invention will be described in more detail based on examples.

### Example 1

### <1> Isolation of hepatic stellate cells

A male Wistar rat (300-350 g in body weight) was anesthetized with ether, 0.5 ml of Nembutal was then intraperitoneally injected, and a portal vein is exposed by performing laparotomy in Cooper. Then, Surflo is inserted into the portal vein slightly from the periphery side.

200 ml of a perfusate (8 g NaCl, 400 mg KCl 88.17 mg NaH₂PO₄·2H₂O, 120.45 mg Na₂HPO₄, 2380 mg HEPES, 350 mg NaHCO₃, and 560 mg CaCl₂·H₂O per litter), 100 ml of a 70-mg pronase perfusate, and 250 ml of a 70 mg collagenase perfusate were circulated. After perfusion, the liver was extracted and then the cells thereof were dispersed. These cells were further digested in a perfusate containing 70 mg pronase, 70 mg collagenase, and 2 mg DNaseI.

After that, the cell dispersion liquid was filtrated through a mesh device and the resulting filtrate was then dispensed into two Falcon tubes, followed by centrifugation at 2000 rpm for 7 minutes. A supernatant was discarded and 0.5 mg DNaseI was then added, and furthermore an agitating solution (8 g NaCl, 370 mg KCl, 210 mg MgCl₂·6H₂O, 70 mg MgSO₄·7H₂O, 150 mg Na₂HPO₄·12H₂O, KH₂PO₄, 991 mg glucose, 227 mg NaHCO₃, and 225 mg CaCl₂ per litter) was added and pipetted, followed by centrifugation at 2000 rpm for 7 minutes. A supernatant was discarded and then 67.5 ml of the agitating solution was added to suspend the cells. Then, a Nicodenz solution (7.75 g Nicodenz was dissolved in 25 ml of solution containing 370 mg KCl, 210 mg MgCl₂·6H₂O, 70 mg MgSO₄·7H₂O, 150 mg Na₂HPO₄·12H₂O, KH₂PO₄, 991 mg glucose, 227 mg NaHCO₃, and 225 mg CaCl₂ per litter) was added and stirred, and then dispersed into centrifuge tubes. In each of the tubes, 1 ml of the agitating solution was layered. Then; it was centrifuged at 3200 rpm for 15 minutes.

A cell layer was obtained at the lower face of the top layer, and then this layer was sucked and transferred to the centrifuge tube. The agitating solution was added to suspend the cells, followed by centrifugation at 2000 rpm for 7 minutes. A precipitate was suspended in 10%FCS-added DMEM and then seeded on a non-coating plastic petri-dish. After 4 hours, a culture solution was replaced with 10%FCS-added DMEM and also cells attached on the petri-dish were defined as hepatic stellate cells in the resting state. Furthermore, the cells attached on the petri-dish after cultured for 3 days and 7 days were provided as hepatic stellate cells at an initial stage of the activation and the activated hepatic stellate cells, respectively.

### <2> Purification of total RNA

For 10⁷ cells, 1 ml of ISOGEN (Nippon Gene Co., Ltd.) was added and homogenized. The obtained homogenate was centrifuged and a supernatant was then recovered. In this supernatant, 200 µl of chloroform was added per milliliter of ISOGEN and gently stirred. After being left to stand for 2 minutes at room temperature, the resultant was centrifuged at 15000 rpm at 4°C for 10 minutes. Then, an aqueous layer was transferred to a new centrifuge tube. Then, an equal amount of 2-propanol was added to the aqueous layer and then left standing at room temperature for 5 minutes, followed by centrifugation at 15000 rpm at 4°C for 15 minutes. The supernatant was discarded and 70% ethanol was added to precipitated pellets, followed by centrifugation at 15000 rpm at 4°C for 15 minutes. Subsequently, 70% ethanol was removed while the pellet was rinsed. Then, the rinsed pellet was dried at room temperature for 5 minutes and DEPC (diethyl pyrocarbonate)-treated water was added to dissolve the pellet. Using 1% agarose gel electrophoresis, it was confirmed that the total RNA fraction thus obtained was purified.

As described above, the total RNA was purified from each of hepatic stellate cells after isolation, after 3 days of incubation, and after 7 days of incubation and then the change of the gene expression amounts were investigated using GeneChip (manufactured by Affymetrix Co., Ltd.).

### <3> Gene expression analysis with GeneChip

The gene expression analysis with GeneChip was carried out in accordance with the protocol recommended by Affymetrix Co., Ltd. The procedures are as follows:

### (1) Probe synthesis

### (i) Double-strand cDNA synthesis

At first, from the total RNA prepared in <2>, a double-stranded cDNA was synthesized using the SUPERSCRIPT Choice System manufactured by Gibco BRL Co., Ltd. 15 µg of the total RNA and 100 pmol of T7- (dT)₂₄ primer were dissolved in a DEPC-treated water so as to be 11 µl in volume. After reacting at 70°C for 10 minutes, it was cooled with ice and then 4 µl of a 5 x 1st strand cDNA buffer (manufactured by Gibco BRL, Co., Ltd.), 2 µl of a 0.1 x DTT (dithiothreitol, manufactured by Gibco BRL, Co., Ltd.), and 1 µl of a 10-mM dNTP mix (manufactured by Gibco BRL, Co., Ltd.) were added and kept at 42°C for 2 minutes. Then, 2 µg of a reverse transcriptase (Superscript II RT) was added therein, followed by reacting at 42°C for 1 hour.

In the reaction solution, the DEPC processing solution, 30 µl of 5 x 2nd strand reaction buffer, 3 µl of 10-mM dNTP, 1 µl of DNA ligase (10 U/µl), 4 µl of DNA polymerase I (10 U/µl), and RNaseH (2 U/µl) were added and mixed, followed by reacting at 16°C for 2 hours. Subsequently, 2 µl of T4 DNA polymerase (5U/µl) was added and reacted at 16°C for 5 minutes. Then, 10 µl of 0.5M EDTA was added therein. In the reaction solution, an equal amount of a (phenol:chloroform = 1:1) solution was added. Then, a tube containing these components was shaken up and down to mix them together. The mixture solution was then subjected to the centrifugation at 15000 rpm at 4°C for 10 minutes. Then, an aqueous layer was transferred into a new centrifuge tube. A 1/10-fold volume of 3M sodium acetate and a 3-fold volume of 100% ethanol were added to the aqueous layer and mixed well. It was left standing at -80° C for 10 minutes, followed by centrifugation at 15000 rpm at 4°C for 10 minutes. The precipitated pellet was rinsed twice with 70% ethanol and dried at room temperature for 5 minutes, followed by adding 12 µl of the DEPC processing solution.

### (ii) Synthesis of biotin-labeled cRNA probe

Next, from the double-stranded cDNA thus synthesized, a biotin-labeled cRNA probe was synthesized using the Bio Array High Yield RNA Transcript Labeling Kit, manufactured by Enzo Co., Ltd. Then, 5 µl of the double-stranded cDNA, 17 µl of the DEPC processing solution, 4 µl of a 10 x HY buffer, 4 µl of a 10 x Biotin labeled ribonucleotides, 4 µl of 10 x DTT, 4 µl of a 10 x RNase inhibitor mix, and 2 µl of 20 x T7 RNA polymerase were mixed together and reacted at 37°C for 4 hours.

Next, from the biotin-labeled cRNA probe solution synthesized as described above, unreacted Biotin labeled ribonucleotides were removed using RNeasy, manufactured by Qiagen Co., Ltd. In the biotin-labeled cRNA probe solution, 160 µl of the DEPC processing solution was added and mixed with 700 µl of RLT buffer, and 500 µl of 100% ethanol was also added and mixed well. A 700-µl aliquot of the solution was added to each of RNeasy mini spin columns and centrifuged at 8000 rpm for 15 seconds. The resulting eluent was added to the RNeasy mini spin column again and centrifuged at 8000 rpm for 15 seconds. Subsequently, 500 µl of RPE buffer was added to the RNeasy mini spin column and then centrifuged at 8000 rpm for 15 seconds. Then, 500 µl of the RPE buffer was added to the RNeasy mini spin column again and centrifuged at 15000 rpm for 2 minutes.

As described above, the RNeasy mini spin column, on which the biotin-labeled cRNA probe was adsorbed, was washed and transferred into a new centrifuge tube. In the RNeasy mini spin column, 30 µl of the DEPC processing solution was added and left standing at room temperature for 1 minute. It was centrifuged at 8000 rpm for 15 seconds, followed by eluting the purified biotin-labeled cRNA probe solution.

Subsequently, the purified biotin-labeled cRNA probe solution was fragmented. The biotin-labeled cRNA probe solution and a 5 x Fragmentation buffer (1/5-fold volume of the final amount of the solution) were mixed and adjusted such that the concentration of the biotin-labeled cRNA probe becomes 0.5 µg/µl, and then reacted at 94°C for 35 minutes. Performing 1% agarose gel electrophoresis, it was confirmed that the probe can be fragmented into fragments each having a length of around 100 base pairs.

### (2) Hybridization

For the hybridization, at first, the results of fragmented biotin-labeled cRNA probes were evaluated using a test chip (Test 2 Chip) to confirm that there was no problem. After that, this examination was performed. In this examination, rat chip sets (RG-U34A, RG-U34B, and RG-U34C) were used. In these three rat chip sets, there were 7000 kinds of known rat genes and 17000 kinds of unknown rat genes in total. Each of the test chip and rat chip set was subjected to hybridization in the following procedures.

60 µg of a fragmented biotin-labeled cRNA probe, 12 µl of control oligonucleotide B2 (5 nM), 12 µl of 100 x control cRNA cocktail, 12 µl of herring sperm DNA (10 mg/ml), 12 µl of acetylated BSA (50 mg/mg), and 600 µl of a 2 x MES hybridization buffer were added and adjusted to 1200 µl in volume with the DEPC processing solution (hereinafter, referred to as a "hybridization cocktail"). The hybridization cocktail was thermally denatured by heating at 99°C for 5 minutes. After standing at 45°C for 5 minutes, the resultant was centrifuged at 15000 rpm at room temperature for 5 minutes. A supernatant was used for hybridization such that a 80-µl aliquot of the supernatant was sampled in the test chip (Test 2 Chip) and a 200-µl aliquot thereof was sampled in the rat chip sets (RG-U34A, RG-U34B, and RG-U34C).

The Gene chip was cooled to room temperatures, and then pre-hybridization was carried out with 1 x MES buffer (80 µl for Test 2 chip and 200 µl for rat chip set) at 60 rpm at 45° C for 10 minutes. Then, the pre-hybridization solution was removed and added with the thermally denatured hybridization cocktail, allowing the hybridization at 60 rpm at 45°C for 16 hours.

### (3) Washing, dyeing, and scanning

The hybridization cocktail was removed from the Genechip and then a non-stringent wash buffer was added therein, followed by washing and dyeing with Fluidic station (manufactured by Affimetrix Co., Ltd.). The washing and dyeing were performed in the Test 2 Chip according to the Fluidic station Mini_euk1 protocol and in the Rat chip set according to the EukGE-WS2 protocol, respectively. After completing the washing and the dyeing, the chip was scanned by a scanner to take in image data.

### (4) Data analysis

The data analysis on the hybridization was performed using the GeneChip analysis suite. The results are listed in Tables 1 to 4, respectively. The expression amount of each gene is represented by an average difference such that the average of the total gene expressions is defined as 100. In the table, the probe set number is denominated by Affymetrix Co., Ltd., which is the administrative number corresponding to each gene. The Unigene is an assembly in which DNA sequences registered in the GenBank are grouped into the category of gene (translation product) species and biological species.

Regarding the column in the table directly after the isolation, hepatic stellate cells were separated and seeded on a non-coating plastic petri-dish. After 4 hours, RNA was prepared from the hepatic stellate cells. The columns on the third day and seventh day correspond to the gene expression levels of cells at the initial stage of activation and activated stellate cells after incubated for 3 days and 7 days, respectively, in the non-coating plastic petri-dish.

There were 105 kinds of known genes having a significant increase in gene expression of the activated hepatic stellate cells incubated on a non-coating plastic petri-dish (increased about 3-fold or more) as compared with the expression level of the hepatic stellate cells in the resting state directly after isolating from the liver in the normal state.

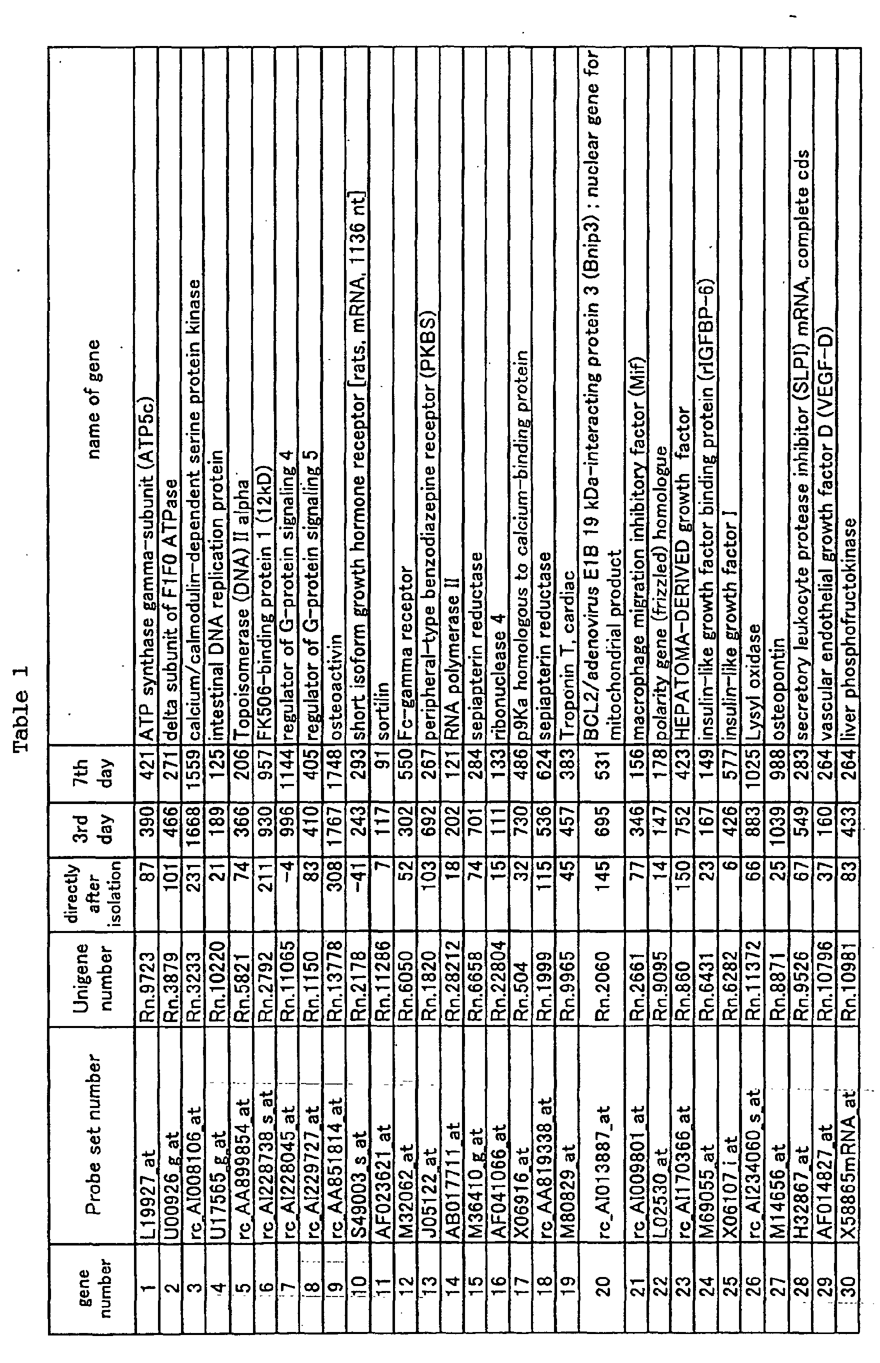

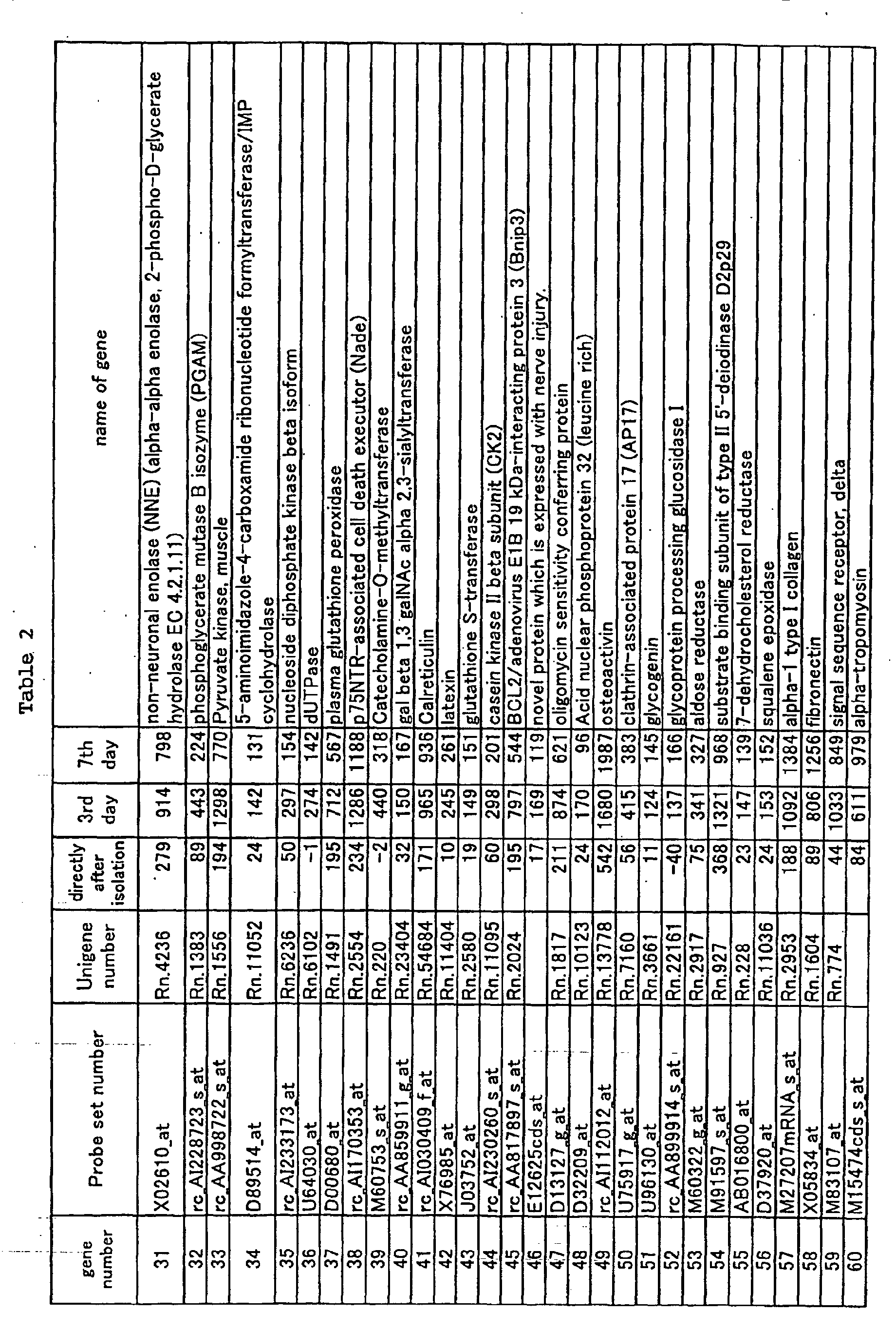

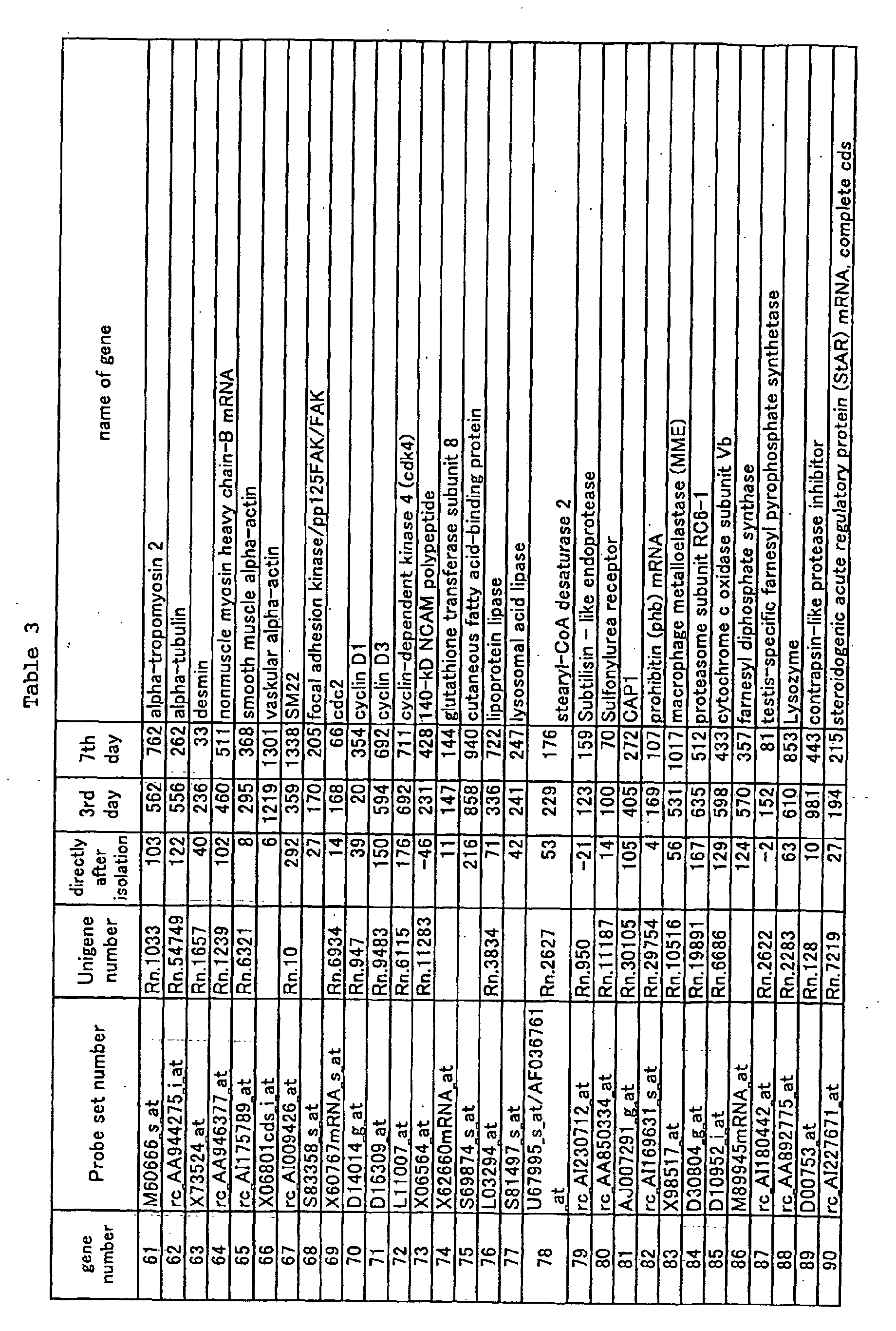

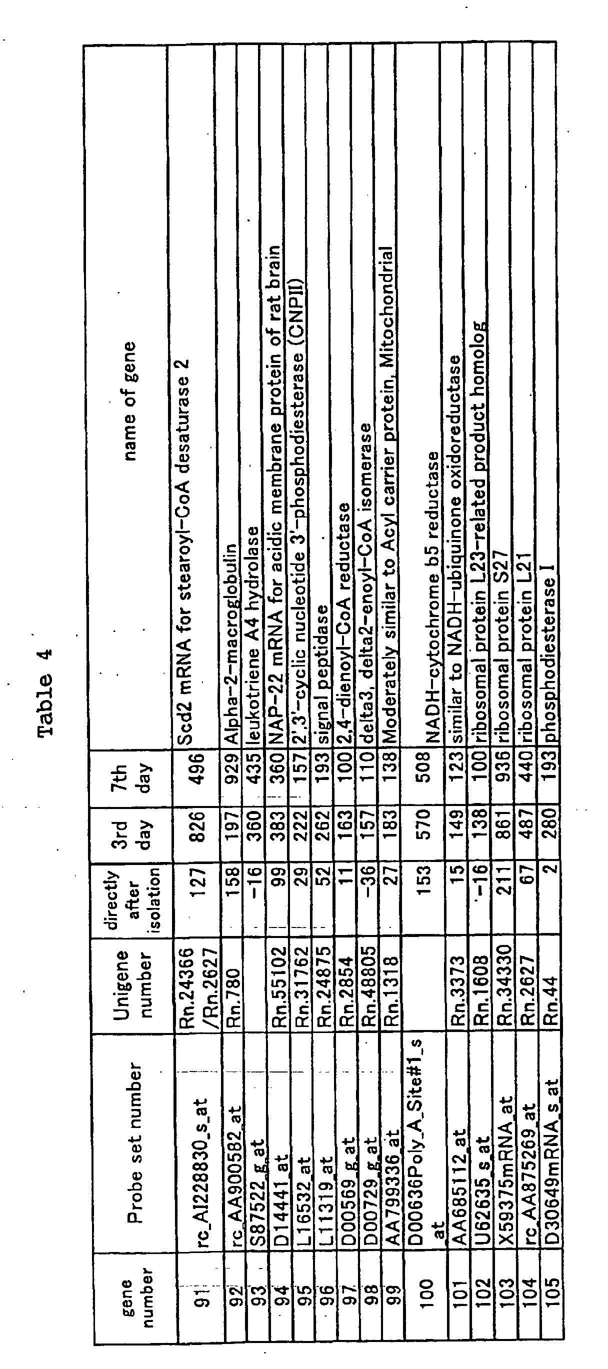

### Example 2

With the method as described in <1> of Example 1, resting-state hepatic stellate cells were prepared and incubated for 24 hours in a MEM medium containing 5% FBS. Then, the medium was replaced with a fresh MEM medium containing 5% FBS or the same medium additionally containing 10 mM cysteine and the stellate cells were further incubated for 48 hours. The medium was removed from the petri-dish. After washing the petri-dish with PBS, cells adhered on the petri-dish were dissolved by Isogen and the RNA thereof was prepared by the method described in <2> of Example 1, followed by the Gene Chip measurement and data analysis by the method described in <3> of Example. The results of the analysis were described as the effects of cysteine to the activated initial hepatic stellate cells.

Similarly, resting-state hepatic stellate cells were prepared and incubated in a MEM medium containing 5% FBS. The incubation was conducted for 3 days, while the medium was replaced with a fresh MEM medium containing 5% FBS every day. After 3 days, it was replaced with a fresh medium that contains 5% FBS or replaced with the same medium additionally containing 10 mM cysteine, followed by incubation for 2 hours or 8 hours. The medium was removed from the petri-dish. After washing the petri-dish with PBS, cells adhered on the petri-dish were dissolved by Isogen and the RNA thereof was prepared, followed by measuring and analyzing the resultant with Gene Chip. The results of the analysis were described as the effects of cysteine to the hepatic stellate cells at the middle stage of activation.

Similarly, resting-state hepatic stellate cells were prepared and incubated in a MEM medium containing 5% FBS. The incubation was conducted for 7 days, while after 3 days and 5 days, the medium was replaced with a fresh MEM medium containing 5% FBS. After 7 days, the medium was replaced with a fresh MEM medium containing 0.1% FBS. After 9 days, it was replaced with a fresh medium that contains 0.1% FBS and PDGF (human recombinant platelet-derived growth factor, manufactured by Sigma Co., Ltd.) at a final concentration of 20 mg/ml or replaced with the same medium additionally containing 10 mM cysteine, followed by incubation for 6 hours or 24 hours. The PDGF was added for the purpose of further accelerating the proliferation of hepatic stellate cells which were decreased in proliferation capacities due to the activation. The medium was removed from the petri-dish. After washing the dish with PBS, cells adhered on the petri-dish were dissolved by Isogen and the RNA thereof was prepared, followed by measuring and analyzing the resultant with Gene Chip. The results of the analysis were described as the effects of cysteine to the hepatic stellate cells at the later stage of activation.

In each of the initial, middle, and later stages of the activation of hepatic stellate cells, the influences of cysteine on the gene expression are shown in Tables 5 to 7. In Tables 5 to 7, the gene numbers are similar to those shown in Tables 1 to 4. Among the genes whose expression levels are increased as the hepatic stellate cells are activated as shown in Tables 1 to 4 in Example 1, the genes where the inhibiting effects of cysteine are observed at each of the initial, middle, and later stages of the activation are shown in Tables 5 to 7. When the expression inhibiting effects are found in the genes at the initial, middle, and later stages of the activation, then the respective genes are evaluated as and the expression intensities (average differences) are illustrated.

Cells incubated for 24 hours after the preparation of hepatic stellate cells were used as hepatic stellate cells at the initial stage of activation. In addition, the measurement value of the cells was described as "initial/initial (24 hr)"; the measurement value of the cells incubated for 48 hours while the medium was replaced with a fresh medium was described as "only medium/initial (+48 hr)"; and the measurement value of cells incubated for 48 hours in the medium added with cysteine was described as "cysteine added/initial (+48 hr)", respectively.

Furthermore, cells incubated for 3 days after the preparation of hepatic stellate cells were used as hepatic stellate cells at the middle stage of activation. In addition, the measurement value of the cells was described as "initial/middle (day 3)"; the measurement value of the cells incubated for 2 hours or 8 hours while the medium was replaced with a fresh medium was described as "only medium/middle (+2 hr)" or "only medium/middle (+8 hr)"; and the measurement value of cells incubated for 2 hours or 8 hours in the medium added with cysteine was described as "cysteine added/middle (+2 hr)" and "cysteine added/middle (+8 hr), respectively.

Furthermore, cells incubated for 9 days after the preparation of hepatic stellate cells were used as hepatic stellate cells at the later stage of activation. In addition, the measurement value of the cells was described as "initial/later (day 9)"; the measurement value of the cells incubated for 6 hours or 24 hours while the medium was replaced with a fresh medium was described as "only medium/later (+6 hr)" or "only medium/later (+24 hr)"; and the measurement value of cells incubated for 6 hours or 24 hours in the medium added with cysteine was described as "cysteine added/later (+6 hr)" and "cysteine added/later (+24 hr), respectively.

As shown in Tables 5 to 7, the cysteine exerted an influence on the expressions of many genes. The cysteine gave effects on the process of activating hepatic stellate cells. Thus, it is predicted that cysteine inhibits hepatic fibrosis. The fact that the cysteine actually inhibits hepatic fibrosis will be described below.

Hepatic fibrosis was induced by intraperitoneally administering 10 mg/kg of dimethylnitrosamine (DMN) to a 6-week SD male rat three times a week for four weeks. Then, an experimental diet containing 0.5% of L-cysteine (Cys) as a subject and that containing 0.5% of casein as a control were fed from the initiation date of the DMN administration. On the 28th day from the initiation of the DNA administration, the liver was sampled from the subject and the amount of hydroxyproline (Hyp) in the liver was measured as an index of hepatic fibrosis using an amino acid analyzer. The results are shown in Fig. 1. As was evident from the figure, an increase in Hyp in the liver, which was increased 6-fold by the administration of DMN, was significantly suppressed by the oral administration of Cys.

As described above, the gene panel of the present invention allows cysteine to inhibit the hepatic fibrosis, where the cysteine has medicinal benefits to change genetic variations required in the process of activating hepatic stellate cells. From the above, it is confirmed that a screening method using this gene panel is an effective screening method.

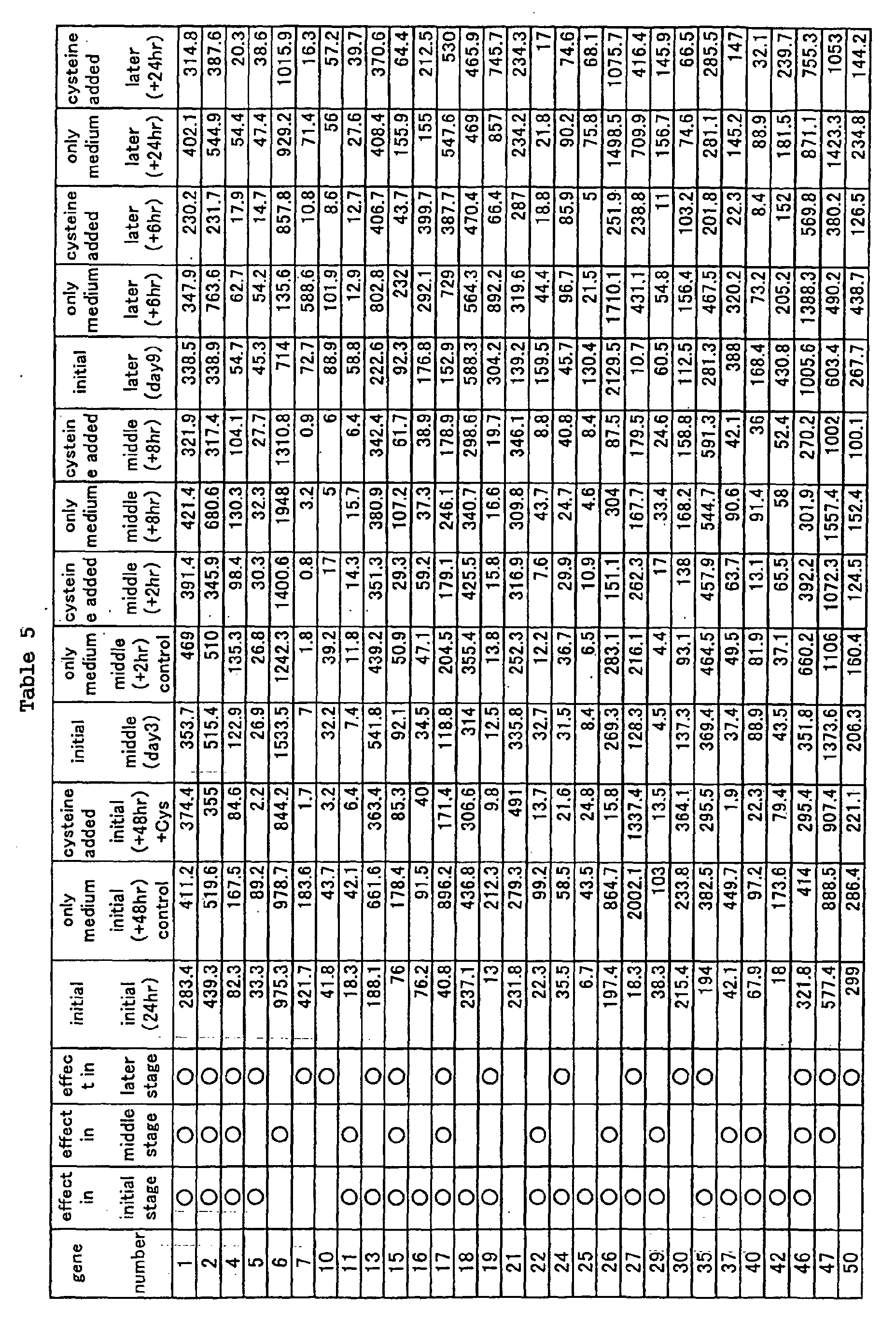

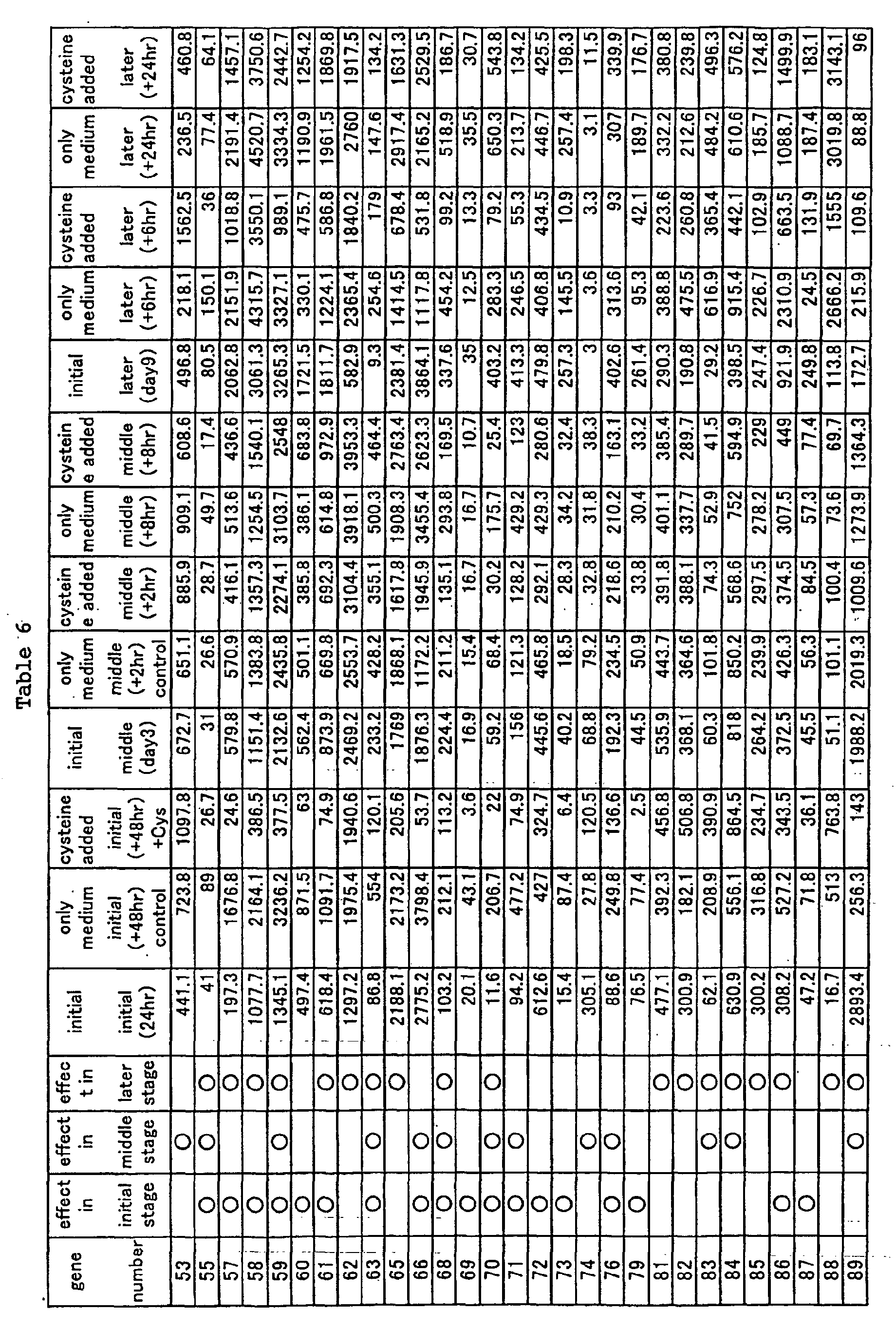

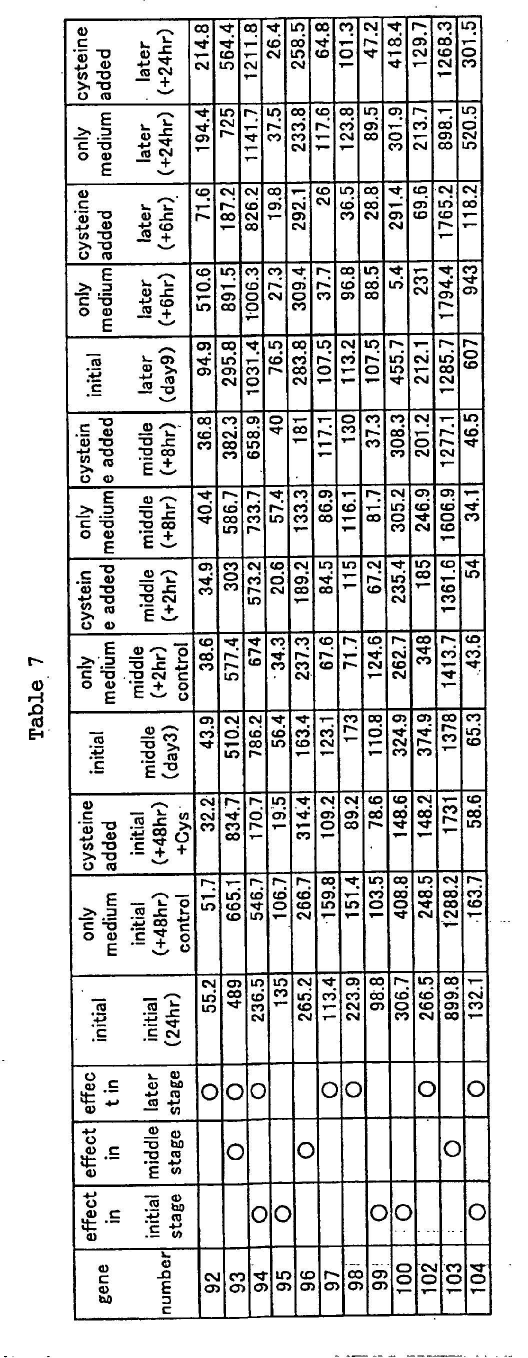

### Industrial Applicability

According to the present invention, there is provided the information about the expression of a gene related to hepatic stellate cells. Using the expression information, the screening of drugs or the like to obtain one capable of inhibiting the activation of the hepatic stellate cells can be carried out.

## Claims

1. A gene panel comprising names and gene expression profiles of genes each showing, in hepatic stellate cells, an increased expression level in an activation state compared with a level in a resting state.

2. A gene panel according to claim 1, wherein the increased expression level of the gene corresponds to a difference of an expression level in a model animal having liver cirrhosis and hepatic fibrosis with an expression level in a normal state in a model animal.

3. A gene panel according to claim 1 or 2, wherein the expression profile comprises a time-varying expression profile in activated hepatic stellate cells.

4. A gene panel according to claim 2 or 3, wherein the model animal is a rat.

5. A gene panel according to any one of claims 1 to 4, further comprising an expression profile of each of at least 5 kinds of genes among 105 kinds of genes represented as Nos. 1 to 105 listed in Tables 1 to 4.

6. A method of producing a gene panel comprising genes each showing, in hepatic stellate cells, an increased expression level in an activation state compared with a level in a resting state, comprising the steps of:
(a) measuring expression levels of various genes in the hepatic stellate cells in the resting state and the expression levels of the genes in the hepatic stellate cells in the activation state;
(b) comparing the expression levels with each other; and
(c) identifying the genes showing the increased expression level in the activation state.

7. A method according to claim 6, wherein the expression levels of the genes in the hepatic stellate cells are analyzed in time course in the step (a).

8. A method according to claim 6 or 7, wherein the expression levels of the gene are analyzed by a gene chip method.

9. A method for screening a drug related to hepatic stellate cell activation, comprising the steps of administering the drug to a model animal or liver tissues or cells, and profiling expressions of genes constituting the gene panel according to claim 1.
